# EUROPEAN PATENT SPECIFICATION

(11) **EP 3 427 659 B1**
(45) Date of publication and mention of the grant of the patent: **03.03.2021**
(21) Application number: 18182617.3
(22) Date of filing: 10.07.2018
(51) Int. Cl.: A61B 5/0404, A61B 5/00, A61B 5/0408

(54) **PORTABLE ELECTROCARDIOGRAPH**
TRAGBARER ELEKTROKARDIOGRAPH
ÉLECTROCARDIOGRAPHE PORTABLE

(30) Priority: 13.07.2017 IT 201700078552
(43) Date of publication of application: 16.01.2019
(73) Proprietor: D-Heart S.r.l., 16149 Genova (IT)
(72) Inventor: BRIANTE, Nicolò, 16149 GENOVA (IT); MAURIZI, Niccolò, 50132 FIRENZE (IT)
(74) Representative: Penza, Giancarlo

(56) References cited:
- WO-A1-2008/157622
- CN-U- 206 102 646
- CN-Y- 2 444 559
- US-A1- 2013 331 720
- US-B1- 6 205 355
- US-B1- 6 605 046

## Description

The present invention relates to a portable electrocardiograph, in particular an electrocardiograph suitable for cooperating with an external electronic device.

Among the different known systems to observe a patient's cardiac activity, the electrocardiogram is the most widespread and cost-effective method. This technique is based on the fact that activity in the heart leads to the generation of electric potential differences, which vary in space and time, and which can be recorded by means of electrodes placed on the surface of the patient's body.

The known electrocardiographs comprise a central unit, a user interface and a series of electrodes that can reach up to ten. The central unit receives the electric signals detected by the electrodes and processes them in order to produce the electrocardiogram trace.

Typically, the user interface comprises a module for entering the commands and the patient's parameters (for example a keyboard) and a module for displaying the results (for example a monitor and/or a printer). Electrocardiographs of the known type are relatively bulky devices, predominantly intended for use in a fixed location, for example in an outpatient department.

Moreover, known electrocardiographs are professional devices, the use of which is reserved to qualified personnel working in the outpatient department.

Such known systems, though widely appreciated, are not without any drawbacks.

For example, because electrocardiographs are intended for use in a fixed location, they usually require to be powered via the power supply network. This means that most known electrocardiographs cannot be used outdoors or in areas where power supply is not reliable, such as in rural areas of emerging and developing countries.

Other drawbacks derive precisely from the fact that known electrocardiographs are intended for professional use. This particular intended use has in fact implied that there has never been a real need to simplify their use. Typically, the management of the complete set of electrodes is rather complex, both as regards the correct positioning of the individual electrodes on the patient, and as regards the handling of the set of wires and electrodes.

The electrodes, whose number can reach up to ten, are connected to the central unit by cable. Each electrode must be positioned at a specific point of the patient's chest and, since each patient has a different chest shape, this operation is rather complex.

In order to avoid inverting the electrodes with each other, a presently adopted solution is differentiating their colours in order to distinguish them. This expedient, however, does not completely solve the problem, because in some cases it is ineffective, for example if the operator is colour blind and/or operates in a poorly lit environment. This second hypothesis is rather frequent, especially in areas where there is no stable power supply. As regards the positioning of the electrodes, a solution is proposed in the patent publication WO 2016/207745, in the name of the same Applicant. As regards the manipulation of the electrodes, the problem that is briefly described below remains unresolved.

Usually from the central unit, by means of a special multipolar connector, a single cable departs, containing the wires of each electrode. At a suitable distance from the central unit, the cable is divided into the individual wires, each one dedicated to a single electrode. The length of the individual wires must be defined keeping in mind the greatest possible distance that may be necessary in use. The measures of the wires are therefore determined on the basis of the anthropometric measurements of patients of high stature and with a wide chest, although they represent a very small percentage. What results is a bundle of rather long and numerous wires, which is therefore subject to the easy formation of tangles.

The possibility, not rare, that a tangle of wires is created lengthens the time necessary to carry out a single electrocardiogram and therefore potentially lengthens the time necessary to obtain a diagnosis. Wire tangles also imply a greater risk of inversion of the electrodes, with consequent alteration of the electrocardiogram.

Moreover, the cable, complete with the whole bundle of wires and electrodes, is rather bulky on the whole, and can be disconnected from the electrocardiograph at the end of use. It is not uncommon for the electrocardiograph and the cable to be stored in different places, for example because the electrodes must be cleaned. In this case, therefore, when a new electrocardiogram has to be performed, a technical time is required to re-assemble the electrocardiograph and the respective electrodes. This technical time could constitute a problem in the case of an emergency.

The separate handling of the cable and the electrode wire bundle increases the risk of the latter becoming dirty and/or prematurely worn. Finally, professional electrocardiographs have a relatively high cost that precludes their private use, even beyond the considerations made above on their difficulty of use.

CN 206 102 646 U discloses an electrocardiograph similar to the one set forth in claim 1 US6605046 (B1) and WO2008/157622 (A1) disclose relevant electrocardiograph monitoring devices configured to be worn hanging around the neck of a subject.

Therefore, the object of the present invention is to overcome the drawbacks highlighted above with respect to the prior art.

In particular, a task of the present invention is to make available a portable electrocardiograph which is light and has a low cost.

Moreover, a task of the present invention is that of making available an electrocardiograph which can be easily used by anyone, even without the aid of qualified personnel for positioning the electrodes on the chest. Furthermore, a task of the present invention is to make available an electrocardiograph which can be used anywhere, even in the absence of a power supply network.

Finally, a task of the present invention is to make available an electrocardiograph which is always operative in a short time.

This object and these tasks are achieved by a portable electrocardiograph according to claim 1 and by its preferred embodiments described in the dependent claims 2 to 10. Further advantages are obtained through an assembly for acquiring an electrocardiogram according to claim 11.

To better understand the invention and appreciate its advantages, some of its exemplifying and non-limiting embodiments are described below with reference to the accompanying drawings, wherein:

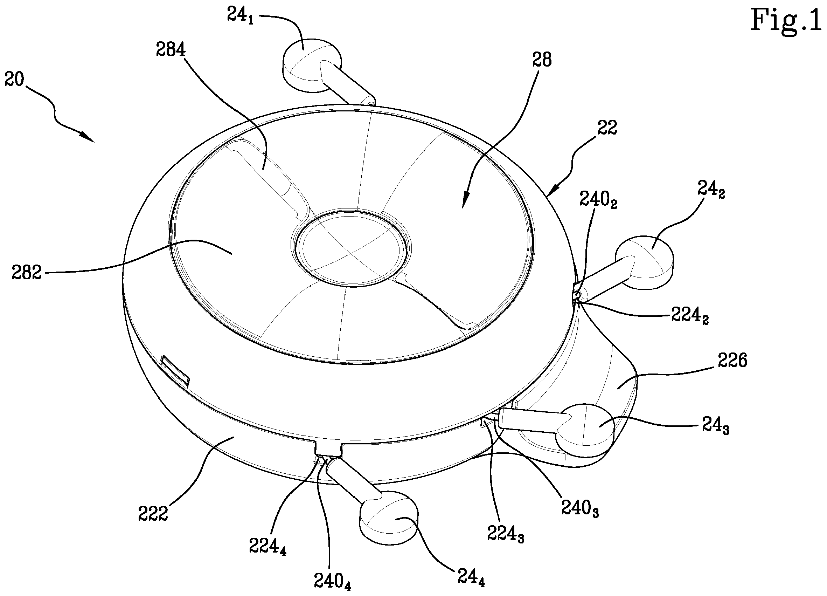

- Figure 1 shows a first perspective view of an electrocardiograph according to an embodiment of the invention;
- Figure 2 shows a second perspective view of the electrocardiograph of Figure 1;

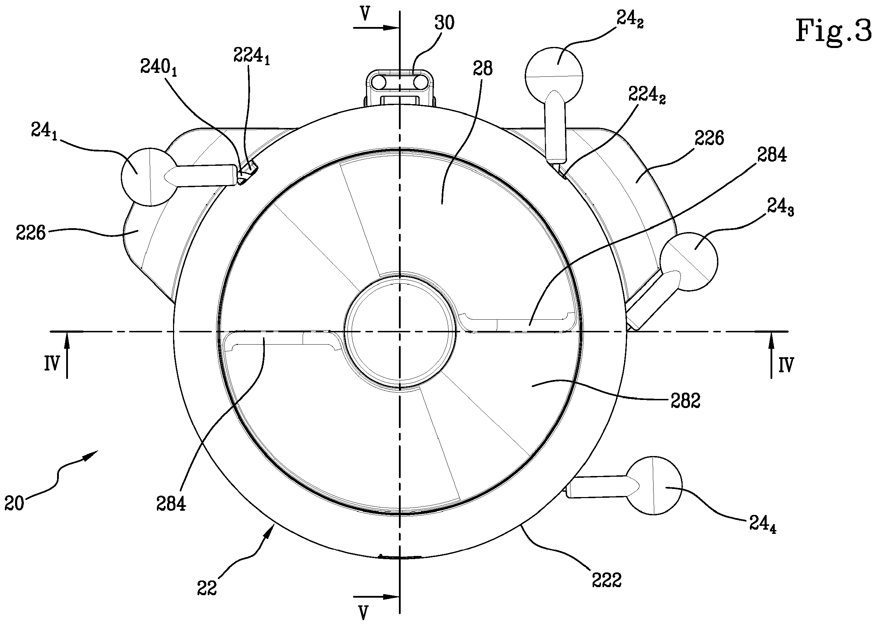

- Figure 3 shows a front view of the electrocardiograph of Figure 1;
- Figure 4 shows a sectional view according to the line IV-IV of Figure 3;
- Figure 5 shows a cross-sectional view according to the line V-V of Figure 3;
- Figure 6 shows a view of the section made along the line VI-VI of Figure 4; and
- Figure 7 schematically shows an assembly for the acquisition of an electrocardiogram comprising an electrocardiograph according to an embodiment of the invention, during use.

In the context of the present discussion, some conventions have been adopted in order to make reading easier and smoother. These terminological conventions are clarified below with reference to the attached figures. When referring to the electrocardiograph in use it is intended that it is used correctly in order to obtain a significant electrocardiogram of the patient's cardiac activity.

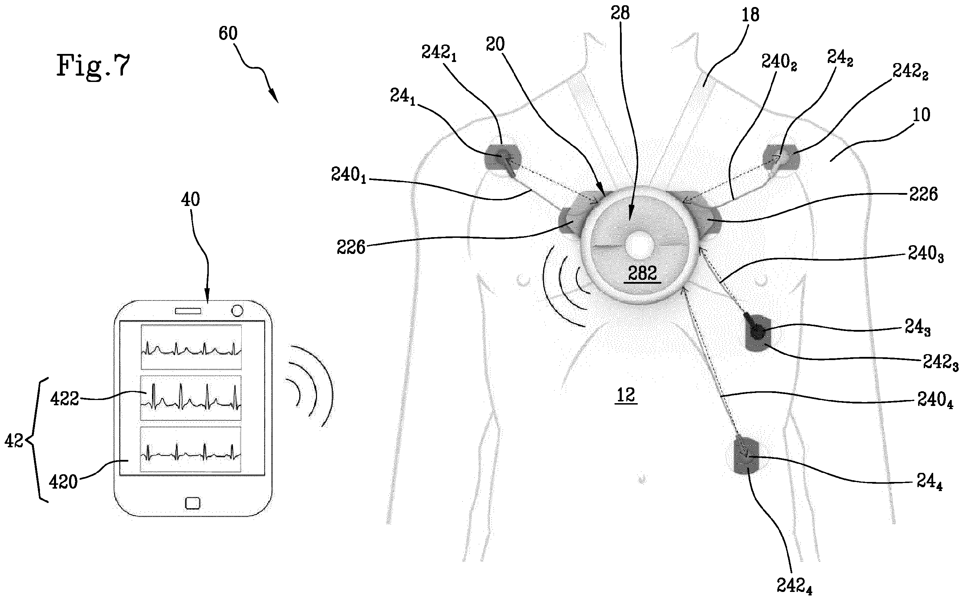

In particular, according to the invention, it is understood that in use the electrocardiograph is hung round the neck of the patient, as can be seen schematically in Figure 7. In particular, it is understood that, according to the correct position of use, the terms "ahead", "forward", "front", and the like, with respect to the terms "behind", "backward", "rear", and the like, are defined unequivocally.

Analogously it is meant that, with respect to the patient 10, the terms "high", "higher", "above" and the like, with respect to the terms "low", "lower", "below" and the like are defined unequivocally.

In the particular embodiment shown in the accompanying figures, a rotation axis X is defined. Hereinafter, the term "axial" means the direction of any straight line parallel to the rotation axis X; the term "radial" means the direction of any half-line having its origin on the rotation axis X and perpendicular thereto; the term "circumferential" means the direction of any circumference having a centre on the rotation axis X and lying in a plane perpendicular to it.

The invention relates to a portable electrocardiograph, indicated as a whole with 20, and suitable for being hung round the neck of a patient 10, for example by means of a band 18.

The electrocardiograph 20 comprises a central body 22, a plurality of connectors 24 and an electronic unit 26.

In the electrocardiograph 20:
- each one of the connectors 24₁, 24₂... is suitable for being associated to a respective electrode 242₁, 242₂... applied on the chest 12 of the patient 10 for detecting respective electric signals;
- each one of the connectors 24₁, 24₂... is suitable for receiving the electric signals detected by the respective electrode 242₁, 242₂... and for transmitting the electric signals to the electronic unit 26;
- the central body 22 comprises a rear surface 220, facing the chest 12 of the patient 10 when the electrocardiograph 20 is in use;
- at least one of the connectors 24n is mounted in a fixed manner on the rear surface 220 of the central body 22;
- each one of the other connectors 24₁, 24₂ is connected to the central body 22 by means of a respective wire 240₁, 240₂ which can be wound and unwound;
- the central body 22 comprises winding/unwinding means 28 suitable for allowing each wire 240₁ to be unwound independently from the other wires 240₂, and for allowing each wire 240₁ to be wound;
- the electronic unit 26 is configured to receive the signals from the connectors 24 and to transmit them to an electronic device 40 external to the electrocardiograph.

Above and below, the reference 24 indicates the plurality of connectors as a whole, while the references 24₁, 24₂... 24ₙ indicate the individual connectors when it is necessary to distinguish them from each other. A similar use is made with the numerical references relating to components that are present in the electrocardiograph 20 in a plurality of specimens.

In a known manner, the connectors 24 are suitable for being associated with respective electrodes 242 applied to the chest 12 of the patient 10 to detect the electric signals generated by the myocardium. The electrodes 242 can assume different embodiments known in themselves. They are preferably disposable and can be applied to the patient's skin so that they remain stable during signal detection. The electrodes 242 can for example be applied by means of an adhesive layer and/or by means of a suction cup.

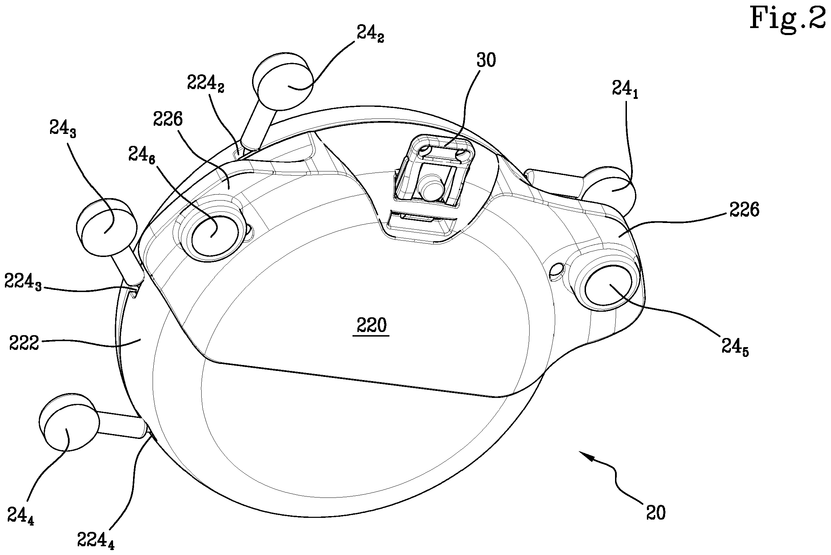

The connectors 24 are also suitable to receive the electric signals detected by the respective electrodes 242 and to transmit these electric signals to the electronic unit 26, in order to obtain the real electrocardiographic trace. The electrodes 242 and the respective connectors 24 may be present in different numbers, depending on the technique used to detect the electric signals generated by the myocardium of the patient 10. According to the embodiment shown in the attached figures, the electrodes 242 and the respective connectors 24 are six. As can be seen in particular in Figure 2, two connectors (denoted by 24₅ and 24₆) are fixedly mounted on the rear surface 220 of the central body 22, while each of the other four connectors (denoted by 24₁, 24₂, 24₃, 24₄) is connected to the central body 22 by means of a respective wire (240₁, 240₂, 240₃, 240₄) which can be wound and unwound.

At least one connector 24ₙ is mounted in a fixed manner on the rear surface 220 of the central body 22. Said connector 24ₙ is arranged in such a way that, when the electrocardiograph 20 is correctly hung round the neck of the patient 10, it is automatically positioned in the correct position for the respective electrode 242ₙ in order to detect the signals. This technical feature is particularly advantageous when the connectors mounted in a fixed manner on the rear surface 220 of the central body 22 are two: the connectors 24₅ and 24₆. In this case, in fact, when the electrocardiograph 20 is correctly hung round the neck of the patient 10, both connectors 24₅ and 24₆ and the respective electrodes 242₅ and 242₆ automatically are placed in the correct position for signal detection, simplifying considerably the step of positioning the electrodes 242 on the chest 12 of the patient 10.

As mentioned above, the winding/unwinding means 28 are suitable for allowing the unwinding of each wire 240₁ completely independently of the other wires 240₂, 240₃... This feature makes it possible to unwind a single wire for a desired length, regardless of the fact that the other wires are unwound or not and possibly of how much they are unwound.

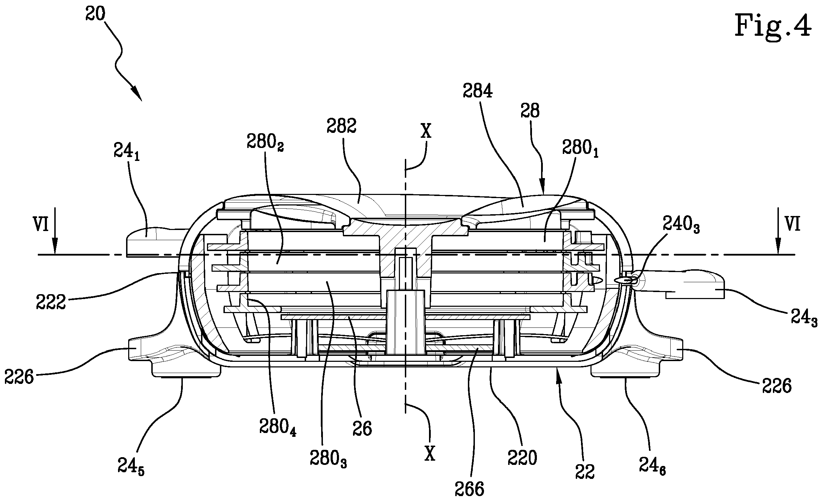

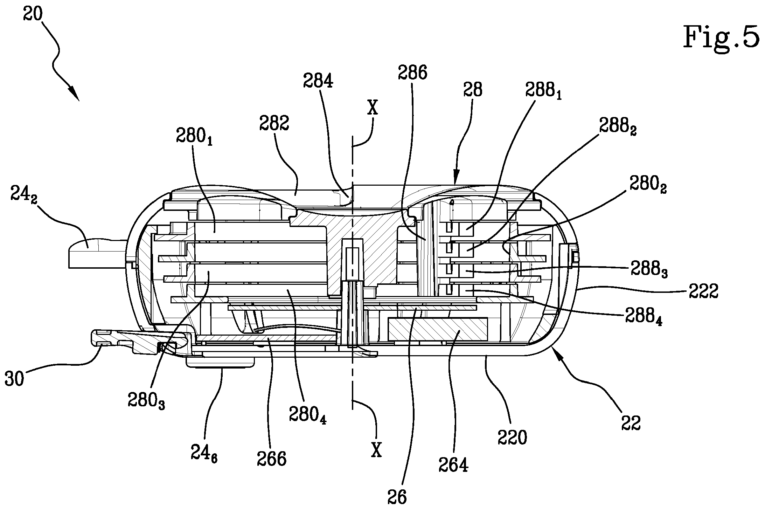

In accordance with the embodiments shown in the attached figures, in particular in the sections of Figures 4 and 5, the winding and/or unwinding means 28 comprise a plurality of drums 280, each of which is rotatably mounted inside the central body 22. In Figures 4 and 5, the wires 240 and other components of the electronic unit 26 have been removed to show more clearly the structure of the winding and/or unwinding means 28. Each of the drums 280₁ is associated with a single wire 240₁ so that, when the drum 280₁ rotates in the central body 22, it allows the unwinding or winding of the respective wire 240₁. In particular, each drum 280₁ can rotate in a first unwinding direction of the respective wire 240₁ or, alternatively, in a second, contrary to the first, winding direction of the respective wire 240₁. Preferably, therefore, the different drums 280 can rotate independently of one another in the unwinding direction of the wire 240.

In the embodiment of the invention illustrated in the attached figures (see in particular the sections of Figure 4 and Figure 5), all the drums 280 share a single rotation axis X. In other embodiments, by virtue of other specific requirements, the drums 280 may have different rotational axes.

Preferably, the winding/unwinding means 28 are suitable for allowing all the wires 240 to be wound at the same time. In this way, it is possible for the user to rewind all the wires 240 with a single action. According to some embodiments, the winding and/or unwinding means 28 comprise a device suitable for rotating all the drums 280 simultaneously in the winding direction.

The device for rotating the drums 280 in the winding direction can be advantageously operated by means of a crown 282 to be rotated manually. Preferably, the crown 282 is shaped in such a way as to allow an effective support of the user's fingers for rotation only in the winding direction of the wires 240. According to the embodiment shown in the accompanying figures, the crown 282 comprises two thrust surfaces 284 which exit the prevailing development plane of the crown 282 and develop in the direction parallel to the rotation axis X. The two thrust surfaces 284 allow the user to easily apply a torque to the crown 282 in the winding direction of the wires 240. At the same time, the crown 282 does not provide an equally convenient support for rotation in the opposite unwinding direction of the wires 240. In fact, the differences in height introduced by the two thrust surfaces 284 are connected by as many ramps which extend with minimum slope for a very wide arch of the crown 282 itself. In accordance with the embodiment of the invention shown in the figures, the unwinding of the wires 240 occurs by directly pulling each individual wire.

In addition or as an alternative to the crown 282, the device for rotating the drums 280 in the winding direction may comprise elastic means which are loaded with the rotation of the drums 280 in the unwinding direction.

In a preferrable embodiment of the invention, the different drums 280 are at least partially constrained to each other when they rotate in the winding direction of the wires 240. In particular, it is preferable that each drum 280₁ rotates together with all the other drums 280₂, 280₃... until the respective wires 240₁ are completely wound.

Since the unwinding length of each individual wire 240₁ may be decided independently of that of the other wires 240₂, 240₃..., it is preferable that the rotation in the winding direction of the different drums 280 is only partially constrained.

In particular, in the embodiment shown in the attached figures, this functionality is obtained in the manner described below.

Each drum 280₁ has a diameter such that the respective wire 240₁ can be completely wound in less than one revolution of the drum 280₁ itself. In other words, the wire 240₁ may extend from the central body 22 for a maximum length included within the circumference of the respective drum 280₁. In this way all the wires 240 can be completely rewound with a maximum of one revolution of the crown 282.

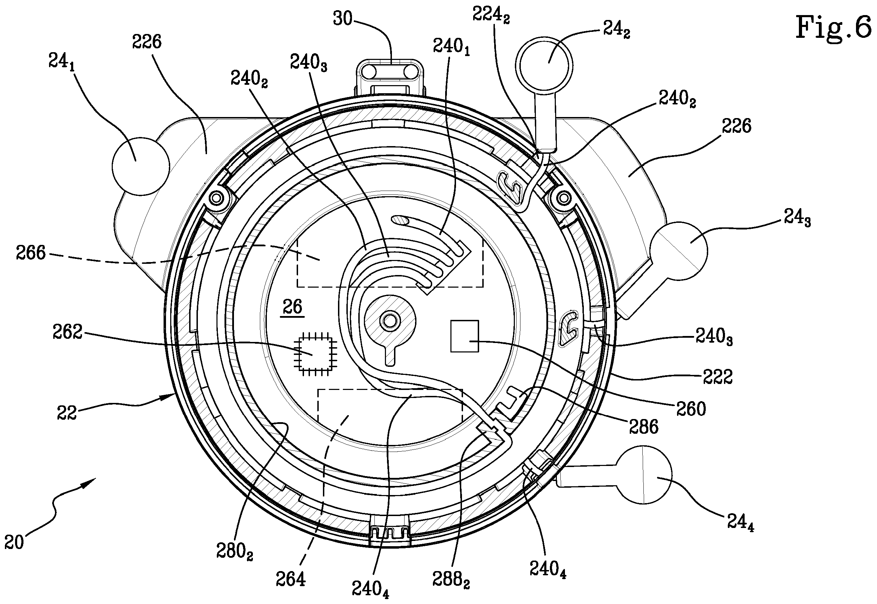

As can be seen in particular in the sections of Figures 5 and 6, the crown 282 comprises, inside the central body 22, a finger 286 which extends parallel to the rotation axis X. Furthermore, the drums 280 each comprise a block 288 extending radially inwardly towards the rotation axis X. The finger 286 of the crown 282 is shaped so as to come into contact in a circumferential direction with the blocks 288 of the drums 280 when the crown 282 rotates.

When the user pulls a first wire 240₁, the first drum 280₁ rotates in the unwinding direction and the relative block 288₁ pushes on the finger 286 also rotating the crown 282. When the user pulls a second wire 240₂ the second drum 280₂ also rotates in the unwinding direction but the relative block 288₂ does not push, at least initially, on the finger 286 because this has already been moved circumferentially by the first block 288₁. The second block 288₂ starts to push on the finger 286 only if the rotation imposed on the second drum 280₂ is greater than the rotation previously imposed on the first drum 280₁. Analogously, it happens with all the wires 240 that are gradually unwound by the user. Therefore, at the end of the unwinding step of the wires 240, the crown 282 will have undergone a rotation equal to the greater rotation between those imposed by the user to the different drums 280. In order to wind the wires, the user acts on the crown 282, for example by means of the thrust surfaces 284, by making it rotate in the winding direction. While the crown 282 rotates, the finger 286 pushes onto the various blocks 288 which it meets gradually, in turn rotating the respective drums 280 in the winding direction. Therefore, the finger 286 begins to push on the drum block which had undergone the major rotation during the unwinding step, and then gradually pushes over the other blocks it encounters. When the finger 286 encounters the drum block that had undergone the minor rotation, all the blocks 288 are aligned and thereafter, until the wires 240 are completely wound, all the drums 280 rotate together.

Preferably, the wires 240 are connected to the electronic unit 26 by means of a fixed coupling. To achieve this, in the embodiment shown in the figures, the electronic unit 26 is arranged radially inside the drums 280. Each of the wires 240 comprises a fixed end on the electronic unit 26 and then extends up to radially cross the wall of the respective drum 280. Preferably each wire 240 passes through the wall of the respective drum 280 at the block 288. As can be seen, for example, in the section of Figure 6, the block 288 can advantageously comprise a labyrinth suitable for blocking the wire 240 with respect to the wall of the drum 280. In this way, the length of the portion of wire 240 radially external to the drum 280 and the length of the portion of wire radially internal to the drum 280 are definitively established.

The portion of wire 240 radially external to the drum 280 is that which is destined to be alternately wound on the drum itself and unwound by the drum itself. Its length must be sufficient for the connector 24 to always reach the respective correctly positioned electrode 24, whatever the size and shape of the chest 12 of the patient 10.

The portion of wire 240 radially internal to the drum 280 extends partially in a radial direction (near the drum 280) and partially in an axial direction (near the electronic unit 26). Since the drum 280 rotates in the central body 22, while the electronic unit 26 is fixed with respect to the central body 22, the portion of wire 240 radially internal to the drum 280 must be sufficiently long to absorb the deformations imposed by the rotation of the drum 280 itself without undergoing damage.

The solution described above allows to obtain the whole electrocardiographic acquisition system without any sliding contact. The sliding contacts are indeed rather delicate because they are structurally exposed to wear and to the formation of oxides and dirt. For these reasons, sliding contacts are often the cause of malfunctions.

According to the invention, the winding/unwinding means 28 wind the wires 240 inside the central body 22. In other words, when the wires 240 are completely wound, preferably they are not reachable from the outside and remain protected inside the central body 22.

The central body 22 comprises a plurality of openings 224 and each of the wires 240₁, 240₂... passes through a respective opening 224₁, 224₂... of the central body 22: in this way, keeping the wires 240 wound inside of the central body 22 and making them slide in the respective openings 224, it is prevented that dirt or foreign bodies may interfere in the winding and unwinding steps of the wires 224.

The fact that each wire 240₁ slides in a respective opening 224₁ allows it to be managed separately from the other wires 224₂, 224₃..., thus preventing them from becoming entangled.

In the embodiments shown in the accompanying figures, the central body 22 of the electrocardiograph 20 further comprises a side wall 222. Preferably the openings 224, through which the wires 240 connecting the connectors 24 pass, are located on the side wall 222 of the central body 22.

Preferably the openings 224₁, 224₂..., through which the wires 240₁, 240₂... pass, are spaced the one from the others. In this particular embodiment, the distance between the openings 224₁, 224₂... keeps the wires separate from each other, further limiting the risk of them becoming entangled.

The openings 224₁, 224₂... are arranged in such a way that, when the electrocardiograph 20 is correctly hung round the neck of the patient 10 as in Figure 7, the openings are oriented in the direction of the correct positioning of the electrode 242₁, 242₂... to be associated with the respective connector 24₁, 24₂...

More particularly, as shown in the accompanying figures, the central body 22 comprises a hook 30 adapted to receive a band 18 for hanging the electrocardiograph 20. The hook 30 is placed in the upper part of the central body 22, in particular above the centre of gravity of the electrocardiograph 20. Preferably, the hook 30 is also centred in the rear part of the central body 22. In this way, when the electrocardiograph 20 is hanging, it spontaneously assumes a predefined position with respect to the chest 12 of the patient 10. The arrangement of the openings 224 is therefore defined on the basis of this predefined position of the electrocardiograph 20 with respect to the chest 12 of the patient 10.

In other words, two openings (indicated in Figure 7 with 224₁ and 224₂) are advantageously directed towards the shoulders of the patient 10, where the first two electrodes 242₁ and 242₂ are to be placed; a third opening 224₃ faces the lower area of the left pectoral of the patient 10, where the third electrode 242₃ is to be placed; the fourth opening 224₄ faces the lower left area of the rib cage of the patient 10, where the fourth electrode 242₃ is to be placed.

The procedure for the correct positioning of the electrodes 242 is not a subject of the present invention. For this purpose, for example, the teachings provided by patent document WO 2016/207745, published in the name of the same Applicant, may be useful. However, the particular arrangement of the openings 224 greatly helps to prevent gross errors during the connection of the connectors 24 to the electrodes 242. By inverting between two connectors any one would in fact obtain an unnatural crossing of the respective wires which would certainly result as a warning to the user.

The possibility to hang the electrocardiograph 20 round the neck of the patient 10 helps to arrange it automatically so that the openings 224 are correctly oriented. However, as the skilled person may well understand, this mode of use is not strictly necessary and the electrocardiograph 20 may also rest against the chest 12 of the patient 10 when the latter is lying down or partially lying down.

In accordance with some embodiments of the invention, the rear surface 220 of the central body 22 of the electrocardiograph 20 comprises at least one flexible wing 226. In this case the at least one connector 24n fixedly mounted on the rear surface 220 of the central body 22 is advantageously mounted on the flexible wing 226.

As already reported above, the connectors fixedly mounted on the rear surface 220 of the central body 22 are advantageously two, conventionally indicated with 24ₙ₋₁ and 24ₙ. In this case, it is preferable that the rear surface 220 of the central body 22 comprises two flexible wings 226 and that each of the two connectors mounted in a fixed manner is mounted on a respective flexible wing 226.

This particular arrangement of the two fixed connectors advantageously allows, thanks to the flexibility of the flexible wings 226, to adapt the electrocardiograph 20 to each specific configuration of the chest 12, for example also in the case of a patient 10 affected by the so-called pigeon chest or *pectus carinatum.*

As already reported above, the electronic unit 26 is configured for receiving the electric signals from the connectors 24 and for transmitting them to an electronic device 40 external to the electrocardiograph 20. The electronic unit 26 preferably comprises a transceiver 260, suitable for receiving the electric signals and transmitting them to the electronic device 40 external to the electrocardiograph 20.

In accordance with some embodiments of the invention, the electronic unit 26 further comprises a processing unit 262 (for example a microprocessor) configured to receive and to process the electric signals from the connectors 24, for example in order to verify the correct placement of the electrodes 242. For this purpose, the processing unit 262 can operate according to a preloaded logic, which exploits, for example, Einthoven's law, as illustrated in publication WO 2016/207745. In accordance with some embodiments, the processing unit 262 is also configured to process the signals received from the connectors 24 in order to make them suitable for the successive steps necessary to produce the electrocardiogram trace; for example, the processing unit 262 can advantageously convert the analog signals provided by the electrodes 242 into digital signals suitable for being transmitted by the transceiver 260.

In accordance with some embodiments of the invention, for example those shown in the attached figures, the electronic unit 26 further comprises a power supply circuit suitable for ensuring the operation of the electrocardiograph even in the absence of a power supply network. The supply circuit preferably comprises a battery 264 and a recharging circuit 266 of the battery. The recharging circuit 266 is preferably suitable for being supplied wirelessly from the outside of the central body 22, for example by means of an induction charging base. This solution advantageously allows to avoid the provision of sockets, pins, connectors or the like on the outer wall of the central body 22.

The object of the present invention is also an assembly 60 for the acquisition of an electrocardiogram, represented schematically in Figure 7. The assembly 60 comprises an electrocardiograph 20 according to any one of the embodiments described above and an electronic device 40, comprising a user interface 42.

Preferably, the user interface 42 of the electronic device 40 comprises a command input module 420 and a display module 422.

The electronic device 40 is preferably of the mobile type, such as a smartphone, a tablet, a PDA, a personal laptop; alternatively, the electronic device 40 is a fixed personal computer or a smart TV.

A considerable advantage offered by the invention is that of exploiting, for the operation of the electrocardiograph 20, the user interface 42 of the electronic device 40: in this way it is possible to contain weights and overall dimensions of the electrocardiograph 20 according to the invention, the components of which are reduced compared to known solutions.

This allows, for example, that the electrocardiograph 20 can be hung round the neck without any safety or comfort problem for the patient 10. Moreover, it is probable that the user already has an electronic device 40 suitable for cooperating with the electrocardiograph 20 in the assembly 60. Preferably, in fact, the transmission of the signals by the electronic unit 26 takes place through standard channels that exploit commonly used protocols. Transmission occurs preferably via Bluetooth technology.

In addition or alternatively, by way of example, the transmission of the signals from the electrocardiograph 20 to the electronic device 40 may take place via cable (for example via a USB cable) or in other wireless modes, in particular by means of short distance radio signals (e.g. via Wi-Fi technology) or through infrared or laser-based technologies.

In this way it is possible to contain the costs and the complexity of the electrocardiograph 20, whose components are reduced compared to the known solutions.

As the skilled person can understand, the invention allows to overcome the drawbacks highlighted above with reference to the prior art.

In particular, the present invention makes available a portable electrocardiograph which, by exploiting the components of an external electronic device, is light and low-cost.

Furthermore, the present invention provides an electrocardiograph which, thanks to its particular structure, can be easily used by anyone, even without the aid of qualified personnel for positioning the electrodes on the chest.

Furthermore, the present invention makes available an electrocardiograph which can be used anywhere, even in the absence of a power supply network.

Finally, the present invention makes available an electrocardiograph which, keeping the wires always organized and protected, is always operative in a short time and prevents the wires themselves from becoming dirty and/or prematurely worn.

It is clear that the specific features are described in relation to various embodiments of the invention with exemplifying and non-limiting intent. Obviously, a person skilled in the art may make further modifications and variations to this invention, in order to meet contingent and specific requirements. For example, the technical features described in connection with an embodiment of the invention may be extrapolated from it and applied to other exemplary constructions of the invention. The scope of protection is defined by the appended claims.

## Claims

1. Portable electrocardiograph (20), suitable for being hung round the neck of a patient (10), comprising a central body (22), a plurality of connectors (24) and an electronic unit (26),
wherein:
- each one of the connectors (24₁, 24₂...) is suitable for being associated to a respective electrode (242₁, 242₂...) applied on the chest (12) of the patient (10) for detecting respective electric signals;
- each one of the connectors (24₁, 24₂...) is suitable for receiving the electric signals detected by the respective electrode (242₁, 242₂...) and for transmitting the electric signals to the electronic unit (26);
- the central body (22) comprises a rear surface (220), facing the chest (12) of the patient (10) when the electrocardiograph (20) is in use;
- at least one of the connectors (24ₙ) is mounted in a fixed manner on the rear surface (220) of the central body (22);
- each one of the other connectors (24₁, 24₂...) is connected to the central body (22) by means of a respective wire 240₁ 240₂... which can be wound and unwound
- the central body (22) comprises a plurality of openings (224₁, 224₂, ...) and each of the wires (240₁, 240₂...) passes through a respective opening (224₁, 224₂...) of the central body (22);
- the electronic unit (26) is configured for receiving the electric signals from the connectors (24) and for transmitting them to an electronic device (40) external to the electrocardiograph;
wherein:
the central body (22) comprises winding/unwinding means (28) suitable for allowing each wire (2401) to be unwound independently from the other wires (2402, 2403...) and for allowing each wire (2401) to be wound; and in that the winding/unwinding means (28) wind up the wires (240) inside the central body (22); wherein the central body (22) comprises a plurality of openings (224) corresponding to the plurality of connectors (24), and wherein each one of the wires (240₁, 240₂...) of the connectors passes through a respective opening (224₁, 224₂...) of the central body (22);
wherein the central body (22) comprises a hook (30) suitable for hanging the electrocardiograph (20), wherein the hook (30) is arranged in such a manner that, when the electrocardiograph (20) is hung round the neck of a patient (10), the electrocardiograph (20) spontaneously assumes a predefined position with respect to the chest (12) of the patient (10); and wherein, when the electrocardiograph (20) is hung around the neck of a patient, it assumes a predefined position with respect to the chest (12) of the patient (10), wherein the arrangement of the openings (224) is defined on the basis of the predefined position of the electrocardiograph (20) with respect to the chest (12) of the patient (10) in such a manner that each opening (224₁, 224₂...) is oriented in the direction of the correct placement of the electrode (242₁, 242₂...) to be associated to the respective connector (24₁, 24₂...);
wherein the openings (224₁, 224₂, ...) are provided on a side wall (222) of the central body (22),
wherein the predefined arrangement of the openings (224₁, 224₂, ...) on the central body (22) is as follows:
- two openings (224₁, 224₂) directed towards the shoulders of the patient (10);
- third opening (224₃) faces the lower area of the left pectoral of the patient (10);
- the fourth opening (224₄) faces the lower left area of the rib cage of the patient (10).

2. Electrocardiograph (20) according to claim 1 wherein the openings (224₁, 224₂...) through which the wires (240₁, 240₂...) pass are spaced the one from the others.

3. Electrocardiograph (20) according to one or more of the preceding claims, wherein the rear surface (220) of the central body (22) comprises at least one flexible wing (226), and wherein the at least one of the connectors (24ₙ) which is mounted in a fixed manner on the rear surface (220) of the central body (22) is mounted on the flexible wing (226).

4. Electrocardiograph (20) according to one or more of the preceding claims, wherein the winding/unwinding means (28) are suitable for allowing all the wires (240) to be wound at the same time.

5. Electrocardiograph (20) according to one or more of the preceding claims, wherein the winding/unwinding means (28) comprise a plurality of drums (280) mounted in a rotatable manner inside the central body (22), each one of the drums (280₁, 280₂...) being associated to a single wire (240₁, 240₂...).

6. Assembly (60) for acquiring an electrocardiogram, the assembly comprising an electrocardiograph (20) according to one or more of the preceding claims and an electronic device (40) external to the electrocardiograph and comprising a user interface (42).

## Patentansprüche

1. Tragbarer Elektrokardiograph (20), geeignet zum Aufhängen um den Hals eines Patienten (10), umfassend einen Zentralkörper (22), eine Vielzahl an Verbindern (24) und eine elektronische Einheit (26), wobei:
- ein jeder der Verbinder (24₁, 24₂ ...) dazu geeignet ist, um mit einer jeweiligen Elektrode (242₁, 242₂ ...) assoziiert zu werden, die an der Brust (12) des Patienten (10) angebracht ist, um jeweilige elektrische Signale zu detektieren;
- ein jeder der Verbinder (24₁, 24₂ ...) zum Empfangen der von der jeweiligen Elektrode (242₁, 242₂ ...) detektierten elektrischen Signale und zum Senden der elektrischen Signale an die elektronische Einheit (26) geeignet ist;
- der Zentralkörper (22) eine Rückseite (220) umfasst, die der Brust (12) des Patienten (10) zugewandt ist, wenn der Elektrokardiograph (20) verwendet wird; mindestens einer der Verbinder (24ₙ) fest an der Rückseite (220) des Zentralkörpers (22) montiert ist;
- ein jeder der anderen Verbinder (24₁, 24₂ ...) über einen jeweiligen Draht (240₁, 240₂ ...), der aufgewickelt und abgewickelt werden kann, mit dem Zentralkörper (22) verbunden ist
- der Zentralkörper (22) eine Vielzahl an Öffnungen (224₁, 224₂, ...) umfasst und ein jeder der Drähte (240₁, 240₂...) durch eine jeweilige Öffnung (224₁, 224₂...) des Zentralkörpers (22) verläuft;
- die elektronische Einheit (26) konfiguriert ist, um die elektrischen Signale von den Verbindern (24) zu empfangen und sie an eine elektronische Vorrichtung (40) außerhalb des Elektrokardiographen zu senden;
wobei:
der Zentralkörper (22) Aufwickel-/Abwickelmittel (28) umfasst, die geeignet sind, um zu ermöglichen, dass ein jeder Draht (240₁) unabhängig von den anderen Drähten (240₂, 240₃ ...) abgewickelt wird, und um zu ermöglichen, dass ein jeder Draht (240₁) aufgewickelt wird; und dadurch dass die Aufwickel-/Abwickelmittel (28) die Drähte (240) innerhalb des Zentralkörpers (22) aufwickeln, wobei der Zentralkörper (22) eine Vielzahl an Öffnungen (224) umfasst, die der Vielzahl von Verbindern (24) entsprechen, und wobei ein jeder der Drähte (240₁, 240₂ ...) der Verbinder durch eine jeweilige Öffnung (224₁, 224₂ ...) des Zentralkörpers (22) verläuft;
wobei der Zentralkörper (22) einen Haken (30) umfasst, der zum Aufhängen des Elektrokardiographen (20) geeignet ist, wobei der Haken (30) so angeordnet ist, dass, wenn der Elektrokardiograph (20) um den Hals eines Patienten (10) aufgehängt wird, der Elektrokardiograph (20) spontan eine vordefinierte Position gegenüber der Brust (12) des Patienten (10) einnimmt; und wobei, wenn der Elektrokardiograph (20) um den Hals eines Patienten aufgehängt wird, er eine vordefinierte Position gegenüber der Brust (12) des Patienten (10) einnimmt, wobei die Anordnung der Öffnungen (224) anhand der vordefinierten Position des Elektrokardiographen (20) gegenüber der Brust (12) des Patienten (10) definiert ist, sodass eine jede Öffnung (224₁, 224₂ ...) in die Richtung der richtigen Platzierung der Elektrode (242₁, 242₂ ...) ausgerichtet ist, die mit dem jeweiligen Verbinder (24₁, 24₂...) assoziiert werden soll; wobei die Öffnungen (224₁, 224₂, ...) an einer Seitenwand (222) des Zentralkörpers (22) bereitgestellt sind,
wobei die vordefinierte Anordnung der Öffnungen (224₁, 224₂, ...) am Zentralkörper (22) wie folgt ist:
- zwei Öffnungen (224₁, 224₂) auf die Schultern des Patienten (10) gerichtet;
- dritte Öffnung (224₃) zum unteren Bereich der linken Brust des Patienten (10) zeigt;
- die vierte Öffnung (224₄) zum unteren linken Bereich des Brustkorbs des Patienten (10) zeigt.

2. Elektrokardiograph (20) nach Anspruch 1, wobei die Öffnungen (224₁, 224₂ ...) durch die die Drähte (240₁, 240₂ ...) verlaufen, von den anderen beabstandet sind.

3. Elektrokardiograph (20) nach einem oder mehreren der vorhergehenden Ansprüche, wobei die Rückseite (220) des Zentralkörpers (22) mindestens einen flexiblen Flügel (226) umfasst und wobei der mindestens eine der Verbinder (24ₙ), der fest auf der Rückseite (220) des Zentralkörpers (22) montiert ist, am flexiblen Flügel (226) montiert ist.

4. Elektrokardiograph (20) nach einem oder mehreren der vorhergehenden Ansprüche, wobei die Aufwickel-/Abwickelmittel (28) geeignet sind, um zu ermöglichen, dass alle Drähte (240) gleichzeitig gewickelt werden.

5. Elektrokardiograph (20) nach einem oder mehreren der vorhergehenden Ansprüche, wobei das Aufwickel-/Abwickelmittel (28) eine Vielzahl an Trommeln (280) umfasst, die drehbar in dem Zentralkörper (22) montiert sind, wobei eine jede der Trommeln (280₁, 280₂ ...) mit einem einzelnen Draht (240₁, 240₂ ...) assoziiert ist.

6. Baugruppe (60) zum Erfassen eines Elektrokardiogramms, wobei die Baugruppe einen Elektrokardiographen (20) nach einem oder mehreren der vorhergehenden Ansprüche und eine elektronische Vorrichtung (40) außerhalb des Elektrokardiographen umfasst und eine Benutzerschnittstelle (42) umfasst.

## Revendications

1. Électrocardiographe portable (20), adapté pour être suspendu autour du cou d'un patient (10), comprenant un corps central (22), une pluralité de connecteurs (24) et une unité électronique (26), dans lequel :
- chacun des connecteurs (24₁, 24₂ ...) est adapté pour être associé à une électrode respective (242₁, 242₂ ...) appliquée sur la poitrine (12) du patient (10) pour détecter des signaux électriques respectifs ;
- chacun des connecteurs (24₁, 24₂ ...) est adapté pour recevoir les signaux électriques détectés par l'électrode respective (242₁, 242₂ ...) et pour transmettre les signaux électriques à l'unité électronique (26) ;
- le corps central (22) comprend une surface arrière (220) faisant face à la poitrine (12) du patient (10) lorsque l'électrocardiographe (20) fonctionne ;
- au moins un des connecteurs (24ₙ) est monté de manière fixe sur la surface arrière (220) du corps central (22) ;
- chacun des autres connecteurs (24₁, 24₂ ...) est connecté au corps central (22) au moyen d'un fil respectif (240₁, 240₂ ...) qui peut être enroulé et déroulé ;
- le corps central (22) comprend une pluralité d'ouvertures (224₁, 224₂, ...) et chacun des fils (240₁, 240₂...) passe à travers une ouverture respective (224₁, 224₂...) du corps central (22) ;
- l'unité électronique (26) est configurée pour recevoir les signaux électriques des connecteurs (24) et pour les transmettre à un dispositif électronique (40) externe à l'électrocardiographe ;
dans lequel :
le corps central (22) comprend des moyens d'enroulement/de déroulement (28) adaptés pour permettre à chaque fil (240₁) d'être déroulé indépendamment des autres fils (240₂, 240₃ ...) et pour permettre à chaque fil (240₁) d'être enroulé ; et
en ce que les moyens d'enroulement/de déroulement (28) enroulent les fils (240) à l'intérieur du corps central (22) ; dans lequel le corps central (22) comprend une pluralité d'ouvertures (224) correspondant à la pluralité de connecteurs (24), et dans lequel chacun des fils (240₁, 240₂ ...) des connecteurs passe à travers une ouverture respective (224₁, 224₂ ...) du corps central (22) ;
dans lequel le corps central (22) comprend un crochet (30) adapté pour suspendre l'électrocardiographe (20), dans lequel le crochet (30) est disposé de telle sorte que, lorsque l'électrocardiographe (20) est suspendu autour du cou d'un patient (10), l'électrocardiographe (20) adopte spontanément une position prédéfinie par rapport à la poitrine (12) du patient (10) ; et dans lequel, lorsque l'électrocardiographe (20) est suspendu autour du cou d'un patient, il adopte une position prédéfinie par rapport à la poitrine (12) du patient (10), dans lequel la disposition des ouvertures (224) est définie sur la base de la position prédéfinie de l'électrocardiographe (20) par rapport à la poitrine (12) du patient (10) de telle manière que chaque ouverture (224₁, 224₂ ...) soit orientée dans la direction du placement correct de l'électrode (242₁, 242₂ ...) à associer au connecteur respectif (24₁, 24₂ ...) ;
dans lequel les ouvertures (224₁, 224₂, ...) sont prévues sur une paroi latérale (222) du corps central (22),
dans lequel la disposition prédéfinie des ouvertures (224₁, 224₂, ...) sur le corps central (22) est la suivante :
- deux ouvertures (224₁, 224₂) dirigées vers les épaules du patient (10) ;
- la troisième ouverture (224₃) fait face à la zone inférieure du pectoral gauche du patient (10) ;
- la quatrième ouverture (224₄) fait face à la zone inférieure gauche de la cage thoracique du patient (10).

2. Électrocardiographe (20) selon la revendication 1, dans lequel les ouvertures (224₁, 224₂ ...), à travers lesquelles passent les fils (240₁, 240₂ ...), sont espacées les unes des autres.

3. Électrocardiographe (20) selon une ou plusieurs des revendications précédentes, dans lequel la surface arrière (220) du corps central (22) comprend au moins une aile flexible (226), et dans lequel l'au moins un des connecteurs (24ₙ), qui est monté de manière fixe sur la surface arrière (220) du corps central (22), est monté sur l'aile flexible (226).

4. Électrocardiographe (20) selon une ou plusieurs des revendications précédentes, dans lequel les moyens d'enroulement/de déroulement (28) sont adaptés pour permettre à tous les fils (240) d'être enroulés en même temps.

5. Électrocardiographe (20) selon une ou plusieurs des revendications précédentes, dans lequel les moyens d'enroulement/de déroulement (28) comprennent une pluralité de tambours (280) montés de manière rotative à l'intérieur du corps central (22), chacun des tambours (280₁, 280₂ ...) étant associé à un seul fil (240₁, 240₂ ...).

6. Ensemble (60) destiné à l'acquisition d'un électrocardiogramme, l'ensemble comprenant un électrocardiographe (20) selon une ou plusieurs des revendications précédentes et un dispositif électronique (40) externe à l'électrocardiographe et comprenant une interface utilisateur (42).
